Europäisches Patentamt

European Patent Office

Office européen des brevets

(19)

(11) Publication number: **0 346 765**
**A2**

## (12) EUROPEAN PATENT APPLICATION

(21) Application number: **89110377.2**

(22) Date of filing: **08.06.89**

(51) Int. Cl.⁴: **C07D 211/22**

(30) Priority: **17.06.88 IT 2100388**

(43) Date of publication of application:
**20.12.89 Bulletin 89/51**

(84) Designated Contracting States:
**AT BE CH DE ES FR GB GR IT LI LU NL SE**

(71) Applicant: **GRUPPO LEPETIT S.P.A.**
**23, Via G. Murat**
**I-20159 Milano(IT)**

(72) Inventor: **Magni, Ambrogio**
**Via Donizetti, 10**
**I-22058 Osnago (Co)(IT)**

(74) Representative: **Sgarbi, Renato et al**
**GRUPPO LEPETIT S.p.A. Patent and**
**Trademark Department 34, Via Roberto**
**Lepetit**
**I-21040 Gerenzano (Varese)(IT)**

(54) **Process for preparing**
**1-(p-tert-butylphenyl)-4-[4'-(alpha-hydroxy-diphenylmethyl)-1'-piperidinyl]butanol.**

(57) The present invention relates to a process for preparing terfenadine which is based on the reduction of terfenadone by an alkali metal borohydride, said process being characterized in that the reaction is carried out in a two-phase system in the presence of a phase transfer catalyst.

The process of the present invention permits obtaining terfenadine in each of the two substantially pure polymorphic forms directly .

EP 0 346 765 A2

# PROCESS FOR PREPARING 1-(p-TERT-BUTYLPHENYL)-4-[4'-(alpha-HYDROXY-DIPHENYLMETHYL)-1'-PYPERIDINYL]BUTANOL

Terfenadine, 1-(p-tert-butylphenyl)-4-[4'-(alpha-hydroxy-diphenylmethyl)-1'-pyperidinyl]butanol is a non-sedating antihistaminic widely used in therapeutical practice.

The preparation of terfenadine is generally carried out by reduction of the corresponding ketone named terfenadone (US Patent No. 3,878,217).

US Patent No. 3,806,526 describes how to prepare said keto compound. The reduction of terfenadone, generally, is preferably carried out by using reducing agents such as sodium borohydride employing as reaction solvents lower alcohols at a temperature ranging from 0°C to the boiling temperature of the mixture.

The reaction may also be carried out by catalytic reduction.

Alternative routes to obtain terfenadine are described, for example, in the following Spanish Patents Nos. 522.610, 537.071, 544.321, 548.555, 539.250 and 547.193.

Terfenadine exists in two distinct polymorphic forms, each form having a different melting point as described in US Patent No. 4,742,175 and in European Patent Application Serial No. 88106898.5 filed on April 29, 1988 with title: "Preparation of polymorphically pure terfenadine".

The higher melting form melts at 149-151°C while the lower melting form melts at 146°C.

The known processes for the preparation of terfenadine give products having variable polymorphic compositions with melting points which are neither constant nor controllable. This makes more difficult the control of large batch productions and the standardization of the operations of formulation in finished pharmaceutical dosage forms.

Therefore, in order to obtain the final product in an established polymorphic form it is necessary to carry out a re-crystallization of the reaction product under pre-established conditions as described in the U.S. patent No. 4,742,175 or in the European Patent application mentioned above.

The present invention relates to a process for preparing terfenadine which is based on the reduction of terfenadone by an alkali metal borohydride, said process being characterized in that the reaction is carried out in a two-phase system in the presence of a phase transfer catalyst.

The process of the present invention permits obtaining terfenadine in each of the two substantially pure polymorphic forms directly.

According to a more detailed description of the present process, a terfenadone solution in an inert organic water unmixable solvent is mixed with an alkaline metal borohydride aqueous solution in the presence of a phase transfer catalyst, i.e. an agent which is able to transfer the borohydride ion from an aqueous phase to an organic phase, maintaining the mixture of the two phases at a temperature varying from 0°C to 90°C, preferably from 40°C to 90°C, for a period of time sufficient to obtain the complete reduction of the keto group of terfenadone (generally from 0.5 to 5 hours).

Usually, the inert organic water unmixable solvent is selected from the aromatic hydrocarbons, preferably benzene, its alkyl derivatives such as toluene, xylene, ethylbenzene, propylbenzene and cumene, cyclohexylbenzene and tetralin.

Among the alkali metal borohydrides, preferably employed are sodium borohydride and lithium borohydride, most preferably, dissolved in an aqueous solution of a diluted alkaline hydroxide.

The amount of borohydride employed ranges from 0.25 to 1 molar proportion for each molar proportion of terfenadone.

The phase transfer catalyst is generally selected from tetraalkylammonium salts wherein the alkyl radicals are selected from the alkyl chains having from 1 to 10 carbon atoms and phenylalkyl chains with an alkyl portion of 1 to 4 carbon atoms with the proviso that, simultaneously, at least one of the alkyl radicals is an alkyl chain of 4 to 10 carbon atoms or a phenylalkyl chain wherein the alkyl portion contains from 1 to 4 carbon atoms and at least one of the alkyl radicals is an alkyl chain of 1 to 4 carbon atoms.

Examples of quaternary ammonium salts which can be useful to carry out the process of the present invention are methyltributylammonium chloride, methyltributylammonium iodide, tetrabutylammonium hydrogen sulphate, methyltrioctylammonium chloride, decyl-trimethylammonium bromide, hexyl-trimethylammonium bromide, benzyltriethylammonium chloride, dibenzyldiethylammonium chloride and the like. The amount of tetraalkylammonium salt employed ranges from 0.01 to 0.05 molar proportions of salt for each molar proportion of terfenadine.

According to a useful method to carry out the process of the invention herein described, terfenadone is dissolved in the selected inert organic water unmixable solvent (for example, xylene) and the appropriate quaternary salt phase transfer catalyst (for example, methyltributylammonium chloride), is added to said solution. A sodium borohydride solution in diluted alkali (for example NaBH$_4$ in NaOH 1N) is slowly added to the above described mixture. Said mixture is kept under stirring at the pre-established temperature and for the appropriate time, which can be determined through preliminary experiments or by checking the conversion rate of terfenadone through analytical tests.

At the end of the reaction, the aqueous phase is separated while the organic phase is washed with water and then worked out for the recovery of the final product.

The conditions of the working out of the organic phase make it possible to obtain either polymorphic form of terfenadine.

If it is desired to obtain the higher melting polymorphic form, the organic phase is reduced to a smaller volume (generally about 1/2 - 1/3 of the starting volume) by partial distillation of the solvent and then the residual mixture is cooled to 0° C - 5° C.

The precipitate so obtained is separated by filtration and then is dried under vacuum, giving the higher melting polymorphic form with good yields and high purity without needing any further recrystallization.

If it is desired to obtain the reaction product as the lower melting polymorphic form of terfenadine, the organic phase, after washing with water, is distilled in order to remove most of the organic solvent. The remaining mixture is then taken up with acetone, ethanol or methanol in a sufficient amount to yield a solution by heating. The solution so obtained is then slowly cooled to about 0° C, yielding a crystalline precipitate. The obtained product corresponds to the substantially pure lower melting polymorphic form of terfenadine.

The process of the present invention besides the advantage of yielding the final product in a substantially pure polymorphic form directly, also permits to use a borohydride amount lower than that required when the procedure is carried out under the conditions described for the original process of the above mentioned U.S. patent, by employing, for example, lower alkanols as solvent medium.

In fact, the alkaline borohydride is much more stable in the aqueous alkaline solutions than in the organic ones (e.g. the lower alkanol solutions); this permits utilizing the reducing agent in a complete way, while, with the use of alcoholic solvents, a part of the reducing agent cannot be effectively utilized because of its instability and this implies a larger consumption of reducing agent for obtaining comparable yields of the final product.

Furthermore, the process of the present invention has the advantage of permitting the use of ready-to-use aqueous solutions of alkali metals borohydrides (in particular, the aqueous solutions of sodium borohydride which are already available on the market), which are stable, instead of preparing every time the borohydride solution in a lower alkanol, which implies handling a rather dangerous solid such as sodium borohydride.

The following examples are merely illustrative without limiting the scope of the present invention:

## Example 1

47.0 Grams of terfenadone base (0.1 mole) are dissolved in 500 ml of xylene, heated to 40° C and added to 0.47 g (0.002 mole) of methyltributylammonium chloride. A solution of 1 g (0.026 mole) of sodium borohydride in 20 ml of 1N sodium hydroxide is slowly added (about 1 h) to the above mixture while maintaining the temperature at about 40° C with external cooling.

The reaction is completed by heating the mixture to 80° C for about 3 hours under stirring. The aqueous phase is separated and the organic phase, after washing with water at pH 7 and decolorizing with charcoal, is concentrated under vacuum at 60° C to a volume of about 250 ml. The mixture is cooled to about 0° C and the crystallized terfenadine is collected by filtration and dried under vacuum at 65° C. Yield 46.1 g of substantially pure higher melting polymorphic form terfenadine (melting point: 151° C).

## Example 2

47 Grams of terfenadone base (0.1 mole) are dissolved in 750 ml of toluene at 50° C. To this solution 0.455 g (0.002 mole) of benzyltrietylammonium chloride are added.

1.89 Grams (0.05 mole) of sodium borohydride in 30 ml of 1N NaOH is slowly added to the above solution while maintaining the temperature at about 50° C under stirring.

The reaction is completed by heating to 80° C until terfenadone disappears (HPLC test).

The reaction mixture is cooled to 50° C and the aqueous phase is separated while the organic phase, after washing with water and decolorizing with charcoal, is concentrated by distillation under vacuum at 50° C to a volume of about 300 ml.

Then, the mixture is slowly cooled to 0° C and the crystallized precipitate is collected by filtration, washed with cold acetone and dried under vacuum at 65° C. Yield 35 g of substantially pure higher melting polymorphic form terfenadine (melting point: 151° C).

## Claims

1. A process for preparing terfenadine by reduction of terfenadone with an alkali metal borohydride characterized in that the reaction is carried out in a two-phase system in the presence of a phase transfer catalyst.

2. A process according to claim 1 wherein the two-phase system comprises:

   a) an inert organic water unmixable solvent containing terfenadone, and

   b) an aqueous alkali metal borohydride solution.

3. A process according to any of claims 1 and 2 wherein:

   a) the inert organic water unmixable solvent is selected from the aromatic hydrocarbons

   b) the aqueous alkali metal borohydride solution consists of sodium borohydride or lithium borohydride dissolved in an aqueous solution of a diluted alkali metal hydroxide and

   c) the phase transfer catalyst is selected from the tetraalkylammonium salts wherein the alkyl radicals are selected from the alkyl chains of 1 to 10 carbon atoms or phenylalkyl with the alkyl portion of 1 to 4 carbon atoms, with the proviso that, simultaneously, at least one of the alkyl radicals is a $(C_4-C_{10})$alkyl chain or a phenylalkyl wherein the alkyl portion contains from 1 to 4 carbon atoms and at least one of the alkyl radicals is an alkyl chain of 1 to 4 carbons atoms.

4. A process as in any of claims 1 to 3 wherein the inert organic solvent is selected from benzene, its alkyl derivatives, cyclohexylbenzene and tetralin.

5. A process as in claim 4 wherein the benzene alkyl derivative is selected from toluene, xylene, ethylbenzene, propylbenzene, and cumene.

6. A process according to any of claims 1 to 5 wherein the inert organic water unmixable solvent is selected from benzene, toluene, xylene, ethylbenzene, propylbenzene, cumene, cyclohexylbenzene or tetralin and the phase transfer catalyst is selected from methyltributylammonium chloride, methyltributylammonium iodide, tetrabutylammonium hydrogen sulphate, methyltrioctylammonium chloride, decyltrimethylammonium bromide, hexyltrimethylammonium bromide, benzyltrietylammonium chloride and dibenzyldiethylammonium chloride.

7. A process according to any of claims 1 to 6 wherein the amount of the alkali metal borohydride ranges between 0.25 and 1 molar proportion for each molar proportion of terfenadone and the amount of the phase transfer catalyst ranges from 0.01 to 0.05 molar proportion for each molar proportion of terfenadone.

8. A process according to any of claims 1 to 7 wherein the reaction temperature ranges between 0°C and 90°C for a time comprised between 0.5 and 5 hours.

9. A process according to claim 8 wherein the inert organic water unmixable solvent is xylene or toluene.

10. A process according to claim 9 wherein the inert organic water unmixable solvent is xylene, the phase transfer catalyst is methyltributylammonium chloride and the alkali metal borohydride solution is sodium borohydride in 1N sodium hydroxide.

11. A process according to claim 10 wherein the inert organic solvent is toluene, the phase transfer catalyst is benzyltriethylammonium chloride and the alkali metal borohydride solution is sodium borohydride in 1N sodium hydroxide.

12. A process according to any of claims 1 to 11 for obtaining terfenadine in the substantially pure higher melting polymorphic form characterized in that at the end of the reaction the organic phase is separated from the aqueous phase, washed with water, reduced to a smaller volume by partial distillation of the solvent and cooled to a temperature ranging from 0°C to 5°C to yield a solid precipitate.

13. A process according to any of claims 1 to 12 for obtaining terfenadine in the substantially pure lower melting polymorphic form characterized in that at the end of the reaction, the organic phase is separated from the aqueous phase, washed with water, distilled in order to remove most of the solvent and then is taken up with a sufficient amount of acetone, methanol, or ethanol to give a solution by heating which is slowly cooled to 0°C to yield a solid precipitate.

14. A process for producing substantially pure high melting polymorph terfenadine comprising:

   (a) contacting a solution of terfenadone in xylene with an aqueous solution of sodium or lithium borohydride in diluted alkali hydroxide at a temperature between 40 and 90°C, in the presence of a phase transfer catalyst selected from methyltributylammonium chloride, methyltributylammonium iodide, tetrabutylammonium hydrogen sulphate, methyltrioctylammonium chloride, decyltrimethylammonium bromide, hexyltrimethylammonium bromide, benzyltrietylammonium chloride and dibenzyldiethylammonium chloride, for a period of time sufficient to complete the reduction reaction,

   (b) separating the organic layer from the aqueous layer,

   (c) washing the organic layer with water at pH 7,

(d) concentrating the organic layer to 1/2 - 1/3 of its original volume and cooling the residual mixture to $0\,^{\circ}C - 5\,^{\circ}C$.